(19)

Europäisches
Patentamt

European
Patent Office

Office européen
des brevets

(11) **EP 2 413 140 A1**

(12) **EUROPEAN PATENT APPLICATION**

(43) Date of publication:
**01.02.2012 Bulletin 2012/05**

(51) Int Cl.:
***G01N 33/50*** *(2006.01)*

(21) Application number: **10305841.8**

(22) Date of filing: **29.07.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS** | (72) Inventor: **The designation of the inventor has not yet been filed** |
| | (74) Representative: **Schneider, Rudolf et al**<br>**Sanofi-Aventis Deutschland GmbH**<br>**Patente Deutschland**<br>**Industriepark Höchst**<br>**Gebäude K 801**<br>**65926 Frankfurt am Main (DE)** |
| (71) Applicant: **SANOFI**<br>**75013 Paris (FR)** | |

(54) **Method for identifying a compound having an antiarrhythmic effect as well as uses relating thereto**

(57)     The present invention relates to a method of identifying a compound having an antiarrhythmic effect and to a use of adenosine-diphospho-ribose cyclase (cardiac ADPRC) for the identification of a compound having said effect.

**Fig. 2**

EP 2 413 140 A1

**Description**

The present invention relates to a method of identifying a compound having an antiarrhythmic effect and to the use of cardiac adenosine-diphospho-ribose cyclase (cardiac ADPRC) for the identification of a compound having said effect.

In the past decades, the treatment of cardiovascular diseases has gained enormous economical and social importance. In fact, in the Western world, cardiovascular diseases by far cover the first position of the list of causes of death by rate. One of the most threatening examples of a cardiovascular disease is represented by cardiac arrhythmia, also known as cardiac dysrhythmia. Cardiac arrhythmia relates to conditions in a subject characterized by showing abnormal electrical activity of the heart. As a result, the heart may beat irregularly or with a retarded or accelerated frequency. Over the past decades, numerous different types and intensities of arrhythmias have been described. Whereas many arrhythmias may be regarded as normal variants in the population, others represent severe diseases or even life-threatening medical emergencies that can result in cardiac arrest and sudden death. Fast rhythms of the heart may be atrial fibrillation, atrial flutter, ventricular tachycardia, and ventricular fibrillation. Remarkably, even in the absence of symptoms, cardiac arrhythmias may predispose the patient to potentially life threatening stroke or embolism. There are different molecular causes that may provoke arrhythmia, what certainly renders the identification of novel agents for diagnostic and/or therapeutic purposes rather difficult.

Due to the relevance to control arrhythmia therapeutically or to detect arrhythmia diagnostically, several antiarrhythmic agents have been developed suppressing fast rhythms of the heart. Often, the antiarrhythmic agents are classified in four groups relating to their molecular target. Class I agents interfere with sodium channels and thereby have an effect on the upstroke of the cardiac action potential, Class II agents interfere with the beta-adrenergic receptors and thereby antagonize catecholamines decreasing sympathetic activity on the heart, Class III agents interfere with potassium channels and thereby prolong repolarization and Class IV agents interfere with calcium channels. Moreover, several other agents may have an antiarrhythmic effect. Adenosine interacts with $A_1$-adenosine receptors and thereby modulates potassium channels. Cardiac glycosides inhibit the myocardial sodium/potassium-ATPase. Parasympatholytic agents block muscarinergic M2- und M4-receptors and sympathomimetic drugs activate myocardial beta1-receptors.

In summary, antiarrhythmic agents known in the art are mainly ion channel blockers with limited selectivity and associated safety issues. They bear severe side effects as the receptors are not entirely heart specific and they may even bear the risk of provoking proarrhythmic effects. Additionally, many agents interact with other drugs. Therefore, there is still a need to develop alternatives to the known antiarrhythmic agents. These alternative agents should not just improve agents targeting to the known targets but should be directed against other molecular target structures such as other ion channels or receptors.

Surprisingly, we found that antagonists of cardiac adenosine-diphospho-ribose cyclase (cardiac ADPRC) also may have an antiarrhythmic effect.

Although, it has recently been demonstrated that bisphenyl compounds may generally inhibit adenosine-diphospho-ribose cyclases (ADPRCs) (WO 08/82169) an antiarrhythmic effect of inhibitors of ADPRCs in general and cardiac ADPRC in particular has not yet been disclosed in the art.

Therefore, it was surprising that in the present invention arrhythmic effects were found for cardiac ADPRC inhibitors. Here, cardiac ADPRC inhibitors were identified by a method based on the measurement of the enzymatic activity of cardiac ADPRC. Samples containing a potential cardiac ADPRC inhibitor were compared with samples without the addition of an inhibitor. Furthermore, these results were confirmed by electrophysiological recordings.

Beside the indication of cardiac arrhythmias cardiac ADPRC inhibitors may be used for a method for treatment or prevention of heart failure, ischemic heart disease and sudden cardiac death in a subject in need thereof and/or for a method for diagnosis of heart failure, ischemic heart disease and/or sudden cardiac death in a subject diseased or potentially diseased or at risk thereof. There is additional evidence that cardiac ADPRC may be involved in the development of pulmonary arterial hypertension, which could be a further indication for cardiac ADPRC inhibitors. Therefore cardiac ADPRC inhibitors may be used for a method for treatment or prevention of pulmonary arterial hypertension in a subject in need thereof and/or for a method for diagnosis of pulmonary arterial hypertension in a subject diseased or potentially diseased or at risk thereof

So far, useful ADPRC inhibitors for the above-mentioned uses were not identified and disclosed in the art as testing the arrhythmic properties of ADPRC in general and testing of antiarrhythmic compounds targeting cardiac ADPRC in particular is still limited due to the absence of suitable test systems and screening assays.

Therefore, the physiological, pathophysiological and biochemical role of adenosine-diphospho-ribose cyclases (ADPRCs) are not yet fully elucidated although they might be highly interesting therapeutic targets and it is therefore desirable to develop, identify and/or characterize agents interacting with them, particularly activating or inactivating the same. Accordingly, method for studying the function and control of adenosine-diphospho-ribose cyclases (ADPRCs) and substances modulating the same are required avoiding or circumventing one or more of the disadvantages of the other drugs is needed. It is desirable that the test method is suitable for high throughput screening in order to allow for testing of e.g. libraries to detect new cardiac ADPRC inhibitors or activators which could be potential therapeutics.

So far, with the methods known in the art, antiarrhythmic effects of cardiac ADPRC inhibitors were not identified due to the absence of suitable methods available for screening and identifying antiarrhythmic cardiac ADPRC inhibitors. Therefore, the development of a novel method for the determination of ADPRC activity in cardiac sarcoplasmatic reticulum membranes and the screening of a compound library unexpectedly led to the detection of antiarrhythmic cardiac ADPRC inhibitors. Surprisingly, a method was found to determine the antiarrhythmic activity of compounds and the conversion of substrates in the presence of active cardiac ADPRC and to identify a compound having an antiarrhythmic effect.

Accordingly, in a first aspect the present invention relates to a method of identifying a compound having an antiarrhythmic effect, the method comprising

-    providing a cardiac adenosine-diphospho-ribose cyclase (cardiac ADPRC),
-    contacting the cardiac ADPRC with a compound, and
-    determining the activity of the cardiac ADPRC in the presence of the compound,

wherein a reduced activity of the ADPCR relative to a control is indicative of the antiarrhythmic effect of the compound.

In general, adenosine-diphospho-ribose cyclases (cardiac ADPRC) catalyze the formation of cyclic adenosine diphosphoribose (cADPR) from nicotinamide adenine dinucleotide (NAD). Moreover, they were also shown to catalyze various other reactions, e.g. cADPR hydrolysis or the formation of nicotinic acid adenine dinucleotide phosphate (NAADP) from nicotinamide adenine dinucleotide phosphate (NADP) through a base exchange reaction. In mammals, two different ADPRCs have been identified: CD38 and CD157. Recently, additional ADPRCs have been isolated from brain, kidney, blood vessels and heart. Interestingly, their properties were found to be different from CD38 and CD157 and the molecular correlates for these activities have not been identified. Cardiac ADPRC is known as an ADPRC found in the heart, particularly in the heart of vertebrates, especially mammals. Methods of isolating cardiac ADPRC are detailed in the present specification and additionally illustrated in the Examples. Cardiac ADPRC has been found to regulate the calcium release from the sarcoplasmatic reticulum of heart cells.

In the context of the present invention, the term "cardiac adenosine-diphospho-ribose cyclase" or "cardiac ADPRC", in particular relates to cardiac ADPRC of mammalian origin, in more particular to cardiac ADPRC of human, pig, rat or guinea pig, in even more particular to cardiac ADPRC of human or pig, most particular to cardiac ADPRC of human or pig heart. In the context of the present invention, the designations "cardiac ADPRC", "adenosine-diphospho-ribose cyclase", "adenosine diphosphoribose cyclase", "adenosine-diphospho-ribosyl cyclase", "adenosine-diphosphate-ribose cyclase", "adenosine-diphosphate-ribosyl cyclase", "ADP-ribosecyclase", "ADP ribose cyclase", , "ADP ribosyl cyclase" and "ADP-ribosyl cyclase" and corresponding designations should be considered as interchangeable.

The term "cardiac ADPRC" may refer to any enzyme isolated from the heart catalyzing the formation of cyclic adenosine diphosphoribose (cADPR) from nicotinamide adenine dinucleotide (NAD).

Cardiac ADPRC may also include all functional derivatives thereof. The term "functional derivative" in the context of cardiac ADPRC refers to proteins with a sequence identity with ACPRC of more than 60%, preferably of more than 80%, more preferably more than 85%, even more preferably more than 90%, even more preferably more than 95%, most preferably more than 97% identity to cardiac ADPRC and at least more than 5%, more than 10%, more than 20%, more than 30%, more than 40%, more than 50%, more than 60%, more than 70%, more than 80%, more than 90%, more than 95%, more than 97% or more that 100 % of the enzymatic activity of the respective wild type protein.

Alternatively or additionally, the cardiac ADPRC or functionalized derivatives thereof may be conjugated, bound or fused to one or more other molecules. These molecules conjugated, bound or fused to cardiac ADPRC may be also designated as "tags". Functional derivatives of cardiac ADPRC maybe understood as cardiac ADPRC conjugated or bound to other molecules that may preferably refer to but may not be limited to cardiac ADPRC conjugated, bound or fused to one or more proteins (e.g. fluorescent proteins such as green fluorescent protein (GFP) or binding proteins such as streptavidin (StpA) or dihyrofolate reductase (DHFR)), one or more lipids or lipidoids, one or more small molecule dyes (e.g. fluorescent dyes such as a Cy dye (e.g. Cy3, Cy5, Cy5), an Alexa dye (e.g. Alexa488, Alexa 546, Alexa 647), S dyes (S0387), fluorescein and functional derivatives thereof (e.g. FITC), rhodamine, HOECHST dye, etc. or UV/Vis dyes, e.g., a p-nitrophenyl moiety, Coomassie Brilliant Blue G-250), one or more quantumdots, one or more binding moieties (e.g., biotin, methotrexate, glycocorticoids or moieties comprising, e.g., one or more active esters, isothiocyanates, maleimids or combinations thereof), one or more soluble and/or insoluble polymers (e.g. polyethylene glycol (PEG), hydroxypropyl methacrylate (HPMA)), functionalized surfaces (e.g. amino reactive or maleimide microarray slides), one or more antibodies or derivatives thereof (e.g. Fab fragments, single chain antibodies, diabodies, triabodies, flexibodies, tandabs), one or more spin labels, one or more enzyme labels (e.g. penicillinase, horseradish peroxidase, alkaline phosphatase), micro- or nanobeads (e.g. functionalized silica beads, sugar-based beads), polymersomes and/or liposomes.

Furthermore, functional derivatives of the cardiac ADPRC may also refer to radioactively labeled cardiac ADPRC, e.g., labeled with $^3$H, $^{32}$P, $^{35}$S, $^4$C or lanthonoids suitable for scintillation assays, computer tomography (CT) or with labels suitable for positron emission tomography (PET). Cardiac ADPRC may be labeled with heavy isotopes, e.g., with $^2$H, $^{13}$C detectable by mass-spectrometry (e.g. ESI-MS or MALDI-MS) or by nuclear magnetic resonance (NMR).

Moreover, functional derivatives of the cardiac ADPRC may also be functional products of posttranslational modifications, functional derivatives of the cardiac ADPRC or functional conjugates of cardiac ADPRC conjugated to other molecules and/or radioactively labeled cardiac ADPRC. Posttranslational modifications are well-known in the art and may comprise but may not be limited to lipidation, phosphorylation, sulfatation, glucosylation, truncation, cyclization of several amino acid moieties, cyclization of the polypeptide strand, oxidation, reduction, decarboxylation, acetylation, amidation, deamidation, disulfide bond formation, pyroglutamate formation, amino acid addition, cofactor addition (e.g. biotinylation, heme addition) and complexation of metal ions, nonmetal ions, peptides or small molecules and addition of iron-sulfide clusters. Evidently, cardiac ADPRC non-covalently bound to co-factors, such as the binding of ATP, ADP, NAD$^+$, NADH+H$^+$, NADP$^+$, NADPH+H$^+$, ions, etc. may also be understood as a functional derivative of cardiac ADPRC irrespective on the biological influence of these co-factors. The cardiac ADPRC may also contain complexed co-factors required for or supporting the enzymatic activity and/or complexed or attached salts, ions and uncharged molecules supporting or inhibiting the cardiac ADPRC or without influence on the enzymatic activity of cardiac ADPRC.

Furthermore, functional derivatives of cardiac ADPRC may also be related to mutated forms of cardiac ADPRC. These mutated forms of cardiac ADPRC or mutated cardiac ADPRC may refer to but may not be limited to cardiac ADPRC encoded by an altered DNA or RNA sequence, a splice variant of cardiac ADPRC or cardiac ADPRC from another species (e.g. viral ADPRC, bacterial ADPRC). An altered DNA or RNA sequence encoding for cardiac ADPRC may occure naturally throughout the population tested or may be of artificial origin. An altered DNA sequence encoding for cardiac ADPRC naturally occurring in the population may be of particular therapeutic interest and may therefore also be of interest in the context of the present invention.

The cardiac ADPRC or functional derivatives thereof may be provided by any means. Herein, the term "providing" may be understood in the widest sense and may include but may not be limited to any biochemical or biotechnological means as known in the art that may be used to obtain cardiac ADPRC. Alternatively, the required amount of cardiac ADPRC may be thawed from a frozen stock and/or may be obtained by a commercial or a noncommercial supplier.

The cardiac ADPRC may be obtained by direct extraction of a sacrificed animal excluding human, by a biopsy sample from a living or dead body from an animal including human, by using tissue from an animal including human not used in a surgical or diagnostic procedure or by cell culture, tissue culture and/or the use of biotechnological techniques.

In case that the cardiac ADPRC is obtained from a sacrificed animal excluding human or by a biopsy sample, the desired organ or tissue, preferably the heart or heart tissue, is explanted and the desired part such as the entire heart, the ventricle and/or the papillar muscle or tissue thereof is/are extracted. The samples may be mounted for methods regarding the action potential. Furthermore, membranes, in particular sarcoplasmatic reticulum membranes, may be extracted from the heart as disclosed by Kranias et al. (Biochim. Biophys. Acta, 709, 28-37, 1982). The extracted biological material may be cleaned of fat and connective tissue and may be homogenized in a suitable buffer that is preferably a cold aqueous buffer, more preferably a cold aqueous buffer of a neutral pH. The buffer may preferably maintain the cardiac ADPRC in its active form and is chosen to avoid, e.g., denaturation of the enzyme and preferably avoids precipitation of the compound(s) and/or the substrate(s). It may be centrifuged and the supernatant may be filtered. Preferably, the sample is centrifuged and filtered several times. The resulting pellet may be resuspended in an aqueous buffer, preferably in an aqueous buffer of neutral pH, preferably maintaining the cardiac ADPRC activity. These centrifugation and resuspension steps may be repeated several times. Finally, a protein concentration suitable for the intended measurements may be adjusted. Preferably, the protein concentration may be between 0.1 and 100 mg/ml, more preferably between 0.5 and 50 mg/ml, even more preferably between 0.5 and 25 mg/ml, most preferably between 5 and 10 mg/ml. Optionally, the suspension may be frozen and stored in the frozen state, preferably at temperatures from -20°C to -80°C.

Optionally, the ADPRC protein may be purified by any means known in the art.

Preferably, the ADPRC protein is purified by a chromatographic method including but not limited to affinity chromatography, ion exchange chromatography, size-exclusion chromatography, HPLC, FPLC and/or UPLC or combinations thereof. More preferred the ADPRC protein is purified by affinity chromatography and/or FPLC. Even more preferred, the used chromatography beads bear a surface fuctionalization suitable for binding the protein selectively. Herein, the term "selectively" does not exclude the binding of closely related proteins and the unspecific binding of other proteins and/or other molecular structures to a minor extent, but may be understood as preferably binding to ADPRC proteins. In this context, the "surface functionalization suitable for binding" may be consisted of immobilized ligands of the ADPRC protein. These ligands may be covalently or uncovalently linked to the surface, preferably they are linked covalently. In this context, the terms "linked" and "conjugated" may be understood exchangeable. Optionally, the ligands are conjugated to the surface of the chromatography bead via a linker as known in the art (e.g. a PEG linker, an alkyl linker, a fatty acid linker, or a peptidic linker). The ligand may be a known or an unknown interaction partner of ADPRC. Preferably, the ligand is a known interaction partner of ADPRC. More preferably, the ligand is nicotinamide adenine dinucleotide (NAD), nicotinamide guanidine dinucleotide (NGD), nicotinamide hypoxanthine dinucleotide (NHD), A001102589 or SW64825.

A crude solution containing the ADPRC protein may be added to a column containing aforementioned beads, the flow rate is adjusted in a way that binding of the ADPRC protein may be accomplished. Then, the column is washed with a suitable buffer solution not disturbing the binding of the ADPRC protein to the beads but diminishing the content of other proteins and/or molecules of other kind. Finally, the ADPRC is eluted with a buffer suitable for eluting the ADPRC protein.

Alternatively, the ADPRC protein may be purified by means of gel electrophoresis, preferably polyacrylamide gel electrophoresis (PAGE), more preferred by PAGE-based electrophoresis preventing the protein from degradation and/or denaturation such as Blue Native Electrophoresis as known in the art. Moreover, specialized electrophoretic methods such as 2D electrophoresis (e.g. 2D-PAGE), capillary electrophoresis (CE) and/or isoelectric focusing (EF) may be used to purify the ADPRC protein.

Alternatively, the ADPRC protein may be purified by precipitation. This may be accomplished by slowly increasing the concentration of additives to a buffer solutions containing dissolved ADPRC protein. Such additives may include but may not be limited to inorganic and organic salts, ethanol, methanol or acetone or mixtures thereof. Alternatively, precipitation may be accomplished by diminishing the salt concentration (e.g. by dialysation of the sample), by heating or cooling the solution or by using shear stress.

Alternatively, the ADPRC protein may be purified by bead precipitation. Here beads bearing bear a surface functionalization suitable for binding the protein selectively are used. Herein, the term "selectively" does not exclude the binding of closely related proteins and the unspecific binding of other proteins and/or other molecular structures to a minor extent, but may be understood as preferably binding activity to ADPRC proteins. In this context, the "surface functionalization suitable for binding" may be consisted of immobilized ligands of the ADPRC protein. These ligands may be covalently or uncovalently linked to the surface, preferably they are linked covalently. In this context, the terms "linked" and "conjugated" may be understood exchangeable. Optionally, the ligands are conjugated to the surface of the chromatography bead via a linker as known in the art (e.g. a PEG linker, an alkyl linker, a fatty acid linker, or a peptidic linker). The ligand may be a known or an unknown interaction partner of ADPRC. Preferably, the ligand is a known interaction partner of ADPRC. More preferably, the ligand is nicotinamide adenine dinucleotide (NAD), nicotinamide guanidine dinucleotide (NGD), nicotinamide hypoxanthine dinucleotide (NHD), A001102589 or SW64825. The beads may be added to a crude solution containing the ADPRC protein. The beads and the protein solution are incubated under conditions enabling the binding of the ADPRC protein to the beads. Subsequently, the bead are washed one or more times. Preferably, washing is performed by centrifugation. Finally, the ADPRC protein is separated from the beads by using a suitable buffer as known in the art.

Optionally, the purification method described above may be combined with each other or with other purification methods known in the art. The solution containing the purified protein may optionally be desalted or the buffer may be changed by any means known in the art (e.g. by dialysis, spin columns, CrossFlow systems, ion exchange chromatography etc.). Furthermore, the protein may be concentrated by means of methods known to those skilled in the art (e.g. spin columns, CrossFlow systems etc.). The salt content of the solution and the protein content my be adjusted for the intended further use.

The purified ADPRC protein may be used in an assay and/or analyzed in an functional assay as specified in the application. Furthermore, the purified ADPRC protein may be used for amino acid sequencing. Numerous methods for amino acid sequencing are well-known to those skilled in the art including but not limited to chemical sequencing methods (e.g.. Edman degradation, Bergmann degradation) mass finger printing (MALDI-MS) or mass spectrometric sequencing (ESI-MS, MALDI-MS). Optionally, the mass spectrometric sequencing is performed by MS-MS or $MS^n$ experiments known to those skilled in the art. Here, the protein or a fragment thereof is ionized (preferably by an ESI or a MALDI ion source). The ions of a certain mass range are selected and isolated. Then the ionized peptide fragments are partly degraded and the degradation products are analyzed. This may be performed once (MS-MS) or n times ($MS^n$). Typically, $MS^n$ sequencing is performed by using an ion trap (IT) or an FTICR system. The amino acid sequence of the proteins or peptide fragments thereof is identified.

The amino acid sequence of the ADPRC protein or sequences of the peptide fragments thereof may be used to find the respective amino acid sequence and/or the nucleic acid sequence (thus, the DNA and/or RNA sequence) in a protein and/or genome databank known to those skilled in the art (e.g. PubMed, SwissProt etc.).

Alternatively, the cardiac ADPRC may be obtained from cell or tissue culture. The cell culture may be originated from the organism of interest, preferably, from the tissue of interest from this organism. Likewise, the tissue culture may be originated from the organism of interest, preferably, from the tissue of interest from this organism. The cells or the tissue may also be competent to express the protein of interest, thus, a functional cardiac ADPRC protein. The cells may be able to proliferate or be unable to proliferate. In the context of the present invention, the term "cell culture" refers to all methods disclosed in the art to cultivate cells *ex vivo*. Thus, the cells may be cultivated in culture medium and at suitable temperature. The culture medium may contain nutrients (e.g. sugars, salts, amino acids and lipids), a buffer system (often comprising one or more chemical buffer substances such as phosphates, hydrogen phosphates and/or Tris with low or no toxicity and/or carbon dioxide ($CO_2$) gassing) and/or optionally one or more antibiotics. Numerous suitable cell culture media are commercially available.

In order to provide sufficient amounts of nutrients and/or to remove metabolites, the medium may be changed when required, preferably on a regular basis. Cells are usually cultured in a suitable atmosphere comprising approximately 5 % (v/v) $CO_2$ and a relatively high relative humidity (e.g. 90 % of the capacity) and a temperature suitable for the cells. The temperature may vary in dependence on the cells (e.g. mammalian and insect cells: ca. 37°C, yeast: ca. 28°C, most bacteria: between 20 and 37°C).

Alternatively, the cardiac ADPRC may be expressed or overexpressed in the cells of interest and/or expressed heterologously. In this context, the term "cell" may refer to a bacterial cell or an eukaryotic cell. The term "overexpressed" refers to conditions in which the expression level of the functional ADDRC is higher compared to the natural expression level in the respected cells. Overexpression may be achieved by the addition of chemical compounds stimulating the expression of the cardiac ADPRC or precursors thereof or by one or more biotechnological methods as known in the art. The term "heterologously expressed" refers to expression in organisms in which the cardiac ADPRC or precursors thereof of the respective species is/are not naturally expressed.

In the context of the present invention, a biotechnological method may relate to any method relying on the use of one or more molecules encoding genetic information. Preferably, the molecule encoding genetic information may be deoxyribonucleic acid (DNA) or ribonucleic acid (RNA). In the context of the present invention term "DNA" also includes functional derivatives or substitutes of DNA (e.g. methylated DNA, peptide nucleic acid (PNA), morpholinos or locked nucleic acids (LNA), DNA conjugated to dyes, proteins, peptides, lipids or to small molecules, etc.). Likewise, in the context of the present invention term "RNA" also includes functional derivatives or substitutes thereof (e.g. methylated RNA, peptide nucleic acid (PNA), morpholinos or locked nucleic acids (LNA), RNA conjugated to dyes, proteins, peptides, lipids or to small molecules, etc.). Most preferably, the molecule encoding genetic information is DNA. The DNA of interest may be obtained from a natural source, in particular extracted from the cells of interest, in more particular heart muscle cells, or can be chemically synthesized. It may be obtained from the genome or from a cDNA library as disclosed in the art and well-known to a person skilled in the art. The obtained DNA may or may not be amplified by one or more biotechnological methods well-known in the art (e.g. polymerase chain reaction (PCR), amplification in bacteria, preferably proliferating bacteria, reverse transcriptase or amplification in eukaryotic cells, preferably proliferating eukaryotic cells).

A nucleic acid encoding for cardiac ADPRC or a precursor thereof, in particular DNA, may be administered to the cell of interest by any means known in the art comprising but not limited to electroporation, complexation with molecules facilitating the uptake (e.g. lipofectamine, cationic lipids or lipidoids, hydrophobe lipids or lipidoids, cationic synthetical polymers (e.g. polyethylene imine (PEI)), hydrophobic polymers, cell-penetrating peptides (CPPs), antimicrobial peptides, protein transduction domains (PTD), polysaccharides (e.g. chitosan), virus-like particles, DNA- or RNA-interacting proteins), conjugation to molecules facilitating the uptake (e.g. lipofectamine, cationic lipids, hydrophobe lipids or lipidoids, cationic synthetical polymers (e.g. polyethylene imine (PEI), cell-penetrating peptides (CPPs), antimicrobial peptides, protein transduction domains (PTD), other transfecting agents or combinations thereof), insertion in viral and/or phage genome of a virus and/or phage that can infect the cell of interest (e.g. a baculovirus, an adenovirus or an vaccina virus), micro- or nanobeads, polymersomes, liposomes, freeze-thaw cycles and/or a gene gun system (e.g. based on gold beads). The nucleic acid, in particular DNA or a functional derivative thereof, may be linear or cyclic (e.g. a plasmid, a bacterial genome or a viral genome).

The molecule encoding the desired genetic information, in particular DNA, may be inserted in the genome of the host cells or may be extrachromosomal (e.g. as a plasmid). The term "host cell" refers to the cell in which the gene is expressed, irrespective whether it is a bacterial cell or an eukaryotic cell and if the cell naturally expresses cardiac ADPRC or does not express cardiac ADPRC. Preferably, a heterogenous host cell may be a bacterial cell or a cell of a cell line. Examples of suitable cell lines include but are not limited to HEK 293, 745-A, A-431, BxPC3, C5N, Caco-2, Capan-1, CC531, CFPAC, CHO, CHO K1, COS-1, COS-7, CV-1, EAHY, EAHY 926, F98, GH3, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, SF9, SK-UT-1, ST, SW 480, SWU-2 OS, U-373, U-937, and Y-1. Other suitable cells are those known to the one of skill in the art.

Preferably, the molecule encoding the desired genetic information, preferably DNA, comprises the gene of interest, a promoter region, a start codon and a stop codon. The genetic information may be expressed permanently or under the control of a repressor and/or a promoter region. The obtained cells may be either used directly or used for tissue cultures or the cells may be harvested and samples comprising cardiac ADPRC are obtained by disrupting the cells. Cells may be disrupted by any means known in the art comprising but not limited to sonication, hypotonic buffer, detergents, UltraTurrax, French press, freeze-thaw cycles, mechanical homogenization and scratching.

Alternatively, DNA or RNA may be used either in cells or in cell-free expression systems such as, e.g., microarray systems in which the DNA or RNA is immobilized and is translated and/or transcribed by the addition of functional cell lysate, comprising the factors required for transcription and/or translation (enzymes, ribosomes, tRNA, amino acids, nucleotides, ATP etc.).

Alternatively, the cardiac ADPRC may also be expressed in a tissue culture. The term "tissue culture" refers to method in which a group of cells forming a three dimensional network is cultivated. The tissue can be formed in culture by the cells themselves or may be formed an extracellular matrix produced by the cells, the culture may be cultivated on a natural or an artificial extracellular matrix (e.g. collagen, elastin, polystyrene, nylon, polylysine) or the tissue may be obtained from an animal including human. The tissue culture may be cultivated in culture medium and at suitable temperature. The culture medium may contain nutrients (e.g. sugars, salts, amino acids, and lipids), a buffer system (often comprising one or more chemical buffer substances such as phosphates, hydrogen phosphates and/or Tris with low or no toxicity and/or carbon dioxide ($CO_2$) gassing) and/or optionally one or more antibiotics. In order to provide sufficient amounts of nutrients and/or to remove metabolites the medium may be changed when required. Optionally, the tissue culture may be perfused with medium. The perfusion may be a permanent or pulsed perfusion.

In summary, the required amounts of cardiac ADPRC may be provided by any means, e.g., (i) by obtaining cardiac ADPRC from a natural source or (ii) by biotechnological methods well-known in the art. The cardiac ADPRC may be in solution, e.g., in a buffer, or in or at a membrane or in a cell, a tissue, an organ or in an organism. Further details on exemplary sources of cardiac ADPRC are given in the examples of the invention. The skilled person will be able to adapt the sourcing of required biological and chemical material in connection with the prevailing method, to the particular test design intended.

Throughout the invention, the term "compound" may refer to any test substance of any chemical nature. The designation "compound" may be understood interchangeable with "agent", "substance" or "chemical substance". It may refer to a small molecule with a molecular weight of less than 500 daltons is size or a salt thereof, a high-molecular weight compound such as a protein, a polypeptide, deoxyribonucleic acid (DNA), ribonucleic acid (RNA), a synthetic polymer, an antibodies and/or antibody derivatives (e.g. Fab fragments, single chain antibodies, diabodies, triabodies, flexibodies, tandabs). The compound may be charged or uncharged. Furthermore, the compound may be hydrophilic, hydrophobic or amphiphilic. The compound may be one or more inorganic salts, one or more salt ions, one or more metals, one or more complexes of one or more inorganic salts or ions and/or a complex of one or more metals, preferably complexed with one or more organic molecules. Alternatively, the compound may be a gas. Preferably, the compound may be soluble in an aqueous buffer or an aqueous solvent. The compound may already have been used as a drug or a medicament. Alternatively, the compound may also be a chemical substance without known therapeutic use or even a substance with unknown chemical properties used in the assay. Furthermore, the term "compound" may also include a substance that may serve as a precursor of a molecule that is used in the method. In particular, this may apply for instable substances that require the synthesis immediately before measuring. Here, a coupled enzyme assay as well-known in the art may apply. Additionally, this may apply for prodrugs as often used in pharmacology, thus, molecules that have to be metabolized before displaying their action (e.g. acetylated, lapidated or oxidized precursors of the active compounds).

Alternatively or additionally, the compound may be conjugated or bound to one or more other molecules, such as small molecules, peptides, proteins, polysaccharides or synthetical polymers. Preferably, it may be conjugated to one or more small molecule dyes (e.g. fluorescent dyes such as a Cy dye (e.g. Cy3, Cy5, Cy5), an Alexa dye (e.g. Alexa488, Alexa 546, Alexa 647), S dyes (e.g. S0387), fluorescein and functional derivatives thereof (e.g. FITC), rhodamine, HOECHST dye, etc. or UV/Vis dyes), one or more quantumdots, one or more binding moieties (e.g., biotin, glycocorticoids or moieties comprising, e.g., one or more active esters, isothiocyanates, maleimids or combinations thereof), one or more soluble and/or insoluble polymers (e.g. polyethylene glycol (PEG), hydroxypropyl methacrylate (HPMA)), one or more spin labels, one or more and/or micro- or nanobeads (e.g. functionalized silica beads, sugar-based beads, polymersomes). Furthermore, the compound may be radioactively labeled, e.g., with $^3$H, $^{32}$P, $^{35}$S, $^{14}$C or lanthonoids suitable for scintillation assays, computer tomography (CT) or with labels suitable for positron emission tomography (PET). Cardiac ADPRC may be labeled with heavy isotopes, e.g., with $^2$H, $^{13}$C detectable by mass-spectrometry (e.g. ESI-MS or MALDI-MS) or by nuclear magnetic resonance (NMR).

As used herein, the term "method" may be understood in the widest sense as an experimental activity or procedure. It may preferably refer to a chemical, biochemical, biological, pharmacological, physiological, electrophysiological, pathophysiological, toxicological, medicinal and/or therapeutic procedure.

As used herein, the term "assay" may be understood in the widest sense, irrespective of its designation as "assay", "test system", "test", "screening", "screening procedure", "screening method", "screening assay", "screening technique", "experimental proceeding", "experimental procedure" or the like. It will be understood as all means that may be used to identify a compound with certain properties or to characterize a certain compound. It may include the testing of one or more samples as well as the screening of libraries containing numerous samples.

When conducting the method, the provided cardiac ADPRC may be contacted with a compound. In the context of the present invention, the term "contacting" may be understood in the widest sense as any means that allow an interaction of the cardiac ADPRC with one or more compounds that may be tested. Preferably, this is achieved by mixing one or more solutions containing the cardiac ADPRC and one or more compounds in a buffer solution suitable for the enzymatic activity of the cardiac ADPRC. The mixing may preferably be accomplished by pipetting, more preferably by mixing two or more solutions of which one contains the cardiac ADPRC and another one contains the compound. In a preferred embodiment, the contacting may be accomplished by automated pipetting.

Preferably, the method may be used to identify a compounds having antiarrhythmic effect. In this context, the term "identifying a compound" means in the widest sense to find a compound showing an effect on the enzymatic activity of the cardiac ADPRC when contacted therewith, in particular a compound showing an inhibitory effect on the enzymatic activity of the cardiac ADPRC when contacted therewith, is found to have an antiarrhythmic effect.

The term "inhibitor" may be understood interchangeable with "antagonist", "inactivator", "inactivating agent", "interacting drug" "compound with inhibitory effect" and "inactivating compound" and other designations for a substance that inactivate enzymatic activity known in the art. Additionally, the test system should use either functional cardiac ADPRC enzyme or molecular compositions comprising functional cardiac ADPRC. The enzyme may be extracted from tissue or from cells or may be heterologously expressed in eukaryotic cells or bacteria cells.

The binding of an inhibitor reduces the activity of the enzyme to which the inhibitor binds. Inhibitor binding is either reversible or irreversible. Irreversible inhibitors usually react with the biomolecule and change it chemically. These inhibitors may e.g. modify key amino acid residues needed for the activity. In contrast, reversible inhibitors bind non-covalently and different types of inhibition are produced depending on whether these inhibitors bind the biomolecule.

If the compound has a specific and significant reducing effect on the cardiac ADPRC, the compound is identified as an inhibitor or ADPRC and, therefore, as a compound having an antiarrhythmic effect. A inhibitor of ADPRC in the context of the present invention means a compound decreasing, cardiac ADPRC enzyme activity in comparison to a control. The person skilled in the art knows statistical procedures to assess whether two values are significantly different from each other such as Student's t-test or chi-square tests.

In a preferred embodiment, the enzyme activity amounts to at most 90 %, preferably at most 75 %, more preferably at most 50 % or 40 %, still more preferably at most 30 % and most preferably at most 20 % of the control.

Preferably, one or more compounds are identified from a group of compounds, preferably from a compound library. As used herein the term "identifying a compound" may be understood as interchangeable with "detection of a compound" or "finding a compound". The term "identifying" herein may be understood as a relative term as evidently to a person skilled in the art a certain threshold has to be applied at which the antiarrhythmic and/or inhibitory effect on the enzymatic activity of cardiac ADPRC is considered strong enough to designate the compound as "identified". It will be understood that the threshold may considerably depend on the experimental setup and may be adjusted depending on the purpose of the method or the intended use of the identified antiarrhythmic compound.

As in the context of the present invention the antiarrhythmic effect is preferably measured as the inhibitory impact of the compound on the enzymatic activity of cardiac ADPRC, the threshold may preferably be given as a certain $IC_{50}$ value. As commonly known in the art, the $IC_{50}$ value refers to the compound concentration required for the half maximal inhibition of the enzymatic activity. Evidently, the $IC_{50}$ value is a relative value dependent on a series of factors including the amount of enzyme, the activation status of the enzyme, the presence of cofactors such as a co-activator or a co-repressor, the presence of activators and inhibitors and the ambient condition such as salt concentration, temperature, pH etc. In the context of the present invention, a compound may preferably be identified as a compound having an antiarrhythmic effect when the $IC_{50}$ value is below 50 $\mu$M, below 20 $\mu$M, below 10 $\mu$M, below 5 $\mu$M, below 4 $\mu$M, below 3 $\mu$M, below 2 $\mu$M, below 1 $\mu$M, below 0.5 $\mu$M, below 0.4 $\mu$M, below 0.3 $\mu$M, below 0.2 $\mu$M, below 0.1 $\mu$M, below 0.05 $\mu$M, below 0.02 $\mu$M or below 0.01 $\mu$M.

The term "antiarrhythmic effect" is used as known in the art. In particular, it describes the suppression of failures regarding the frequency or the regularity of the rhythm of the heart named cardiac arrhythmias or other effects resulting from abnormal electrical activity of the heart. Cardiac arrhythmias play an important role in several diseases, such as, e.g., ventricular tachycardia, ventricular fibrillation, atrial fibrillation and atrial flutter.

The antiarrhythmic effect of a compound may be measured by determining the activity of the cardiac adenosine-diphospho-ribose cyclase (cardiac ADPRC). The results may be confirmed by electrophysiological recordings of heart cells, heart tissues or heart.

Throughout the invention, the term "determining the activity" may be understood in the widest sense as any means that may be used to characterize the functionality of the cardiac ADPRC. Preferably, it is related to the characterization of the enzymatic activity of the cardiac ADPRC. The enzymatic activity may be related to the catalytic activity regarding the formation of cyclic adenosine diphosphoribose (cADPR) from nicotinamide adenine dinucleotide (NAD) or the catalytic activity regarding the metabolism of surrogates such as NAD and/or cADPR, respectively. Preferably, the ADPRC shows reduced enzymatic activity upon incubation with an inhibitory compound. Exemplary, the determination of the enzymatic activity is shown in the examples of the invention. The term "surrogate" may be understood in the widest sense as a molecule that can be metabolized and/or enzymatically converted in stead of the natural substrate(s) such as nicotinamide adenine dinucleotide (NAD).

In order to detect the antiarrhythmic effect of a compound, the activity of the cardiac ADPRC may be determined in the presence of a compound relative to a control. The term "control" herein may refer to a positive control, thus, a complete sample without compound that will result in a comparably high conversion rate of the substrate. Upon addition of a compound bearing an inhibitory effect, the sample comprising the compound may show a conversion rate lower than the control. Additionally, the background, therefore, the conversion of the substrate independent on the ADPRC activity, may be measured by means of a sample containing the substrate in the respective buffer lacking the enzyme. In this background sample, there will be no or nearly no conversion of the substrate. It will be understood by a person skilled in the art that the composition of the control and the background sample, respectively, may vary according to the experimental setup. Moreover, the experimental setup disclosed in the present invention may be understood as an exemplary setup but should not limit the scope of the invention.

Furthermore, the method of the invention may be used in order to elucidate the function and activity of cardiac adenosine-diphospho-ribose cyclase (cardiac ADPRC), in particular in the presence of compounds interacting with it, in more particular in the presence of compounds inhibiting the enzymatic activity. It may be used for enzyme kinetic studies. A compound inhibiting the enzymatic activity may act directly by interacting with the cardiac ADPRC by itself or may act indirectly by interacting with molecular cofactors of the cardiac ADPRC or by modifying the level of cardiac ADPRC at the site of pharmacological action. The interaction between the compound with its molecular target or its molecular targets, in particular with cardiac ADPRC may be a covalent or a non-covalent or a complex bond, it may be uncompetitive, competitive, non-competitive or allosteric. In this context, the site of pharmacological action may be a tissue or a cell, in particular a cardiac cell or tissue, in more particular a cardiac muscle cell or tissue or an intracellular compartment of said cell, in particular an intracellular compartment storing calcium, in more particular a sarcoplasmic reticulum or a membrane, in particular a sarcoplasmic reticulum membrane. The compound may be enriched at or depleted from the site of pharmacological action. Furthermore, the method may be used to develop, identify and/or characterize compounds interacting with them, particularly activating or inactivating the same, more in particular inactivating the same. Moreover, the method may be used to determine the cardiac ADPRC concentration or the enzymatic activity of cardiac ADPRC in a tissue. Moreover, the method may be used to determine mutated forms of cardiac ADPRC. Moreover, the method may be used to characterize the properties of cardiac ADPRC and/or of mutated forms of cardiac ADPRC.

In a preferred embodiment, the cardiac ADPRC is an intracellular membrane-bound cardiac ADPRC, particularly a sarcoplasmatic reticulum membrane-bound cardiac ADPRC.

In the context of the present invention the term "membrane-bound" means that the cardiac ADPRC is either integrated in a biological membrane or attached to a biological membrane. A biological membrane is a lipid bilayer as known by a person skilled in the art. Alternatively, the cardiac ADPRC may also be integrated in or attached to an artificial membrane composed of lipids, phospholipids, steroids or organic compounds or combinations thereof or the cardiac ADPRC may also be integrated in or attached to micelle, to a liposome or a polymersome membrane or surface.

The term "sarcoplasmatic reticulum" may be understood as generally known in the art. Thus, it may be understood as an organelle that is typically found in muscle cells and facilitates the storage of calcium ions and thereby plays an important role in muscle contraction.

In a preferred embodiment, the cardiac ADPRC is of mammalian origin, in particular from human, pig, guinea pig or rat, especially pig or human heart.

In this context, the term "origin" merely relates to the natural source, where the cardiac ADPRC is originated from, *id est,* where the sequence of the used polypeptide is derived from. It should not be understood as limited to the substantial and material source of the cardiac ADPRC. Moreover, the cardiac ADPRC may alternatively be also isolated from mammalian cells and/or tissues of other species, but may also be expressed in bacteria or eukaryotic cell culture or eukaryotic tissue culture.

A series of test designs is known in the art to which the method of the present invention may be adapted. Further details on exemplary tests are given in the Examples. The test may be a heterogeneous or homogeneous assay. As used herein, a heterogeneous assay is an assay which includes one or more washing steps, whereas in a homogeneous assay such washing steps are not necessary and the reagents and compounds are only mixed and measured. The skilled person will be able to adapt the test system, e.g., by adding further agents required in connection with the prevailing method, to the particular test design intended.

In accordance with the present invention, the assay is designed to determine the enzymatic activity of cardiac ADPRC or derivatives thereof. As mentioned above, cardiac ADPRC is an enzyme catalyzing the formation of cyclic adenosine diphosphoribose (cADPR) from nicotinamide adenine dinucleotide (NAD) or cyclic guanidine diphosphoribose (cGDPR) from nicotinamide guanidine dinucleotide (NGD) or cyclic inosine diphosphoribose (cIDPR) from nicotinamide hypoxanthine dinucleotide (NHD). Accordingly, the enzyme activity may be measured as reduction of the amount of substrate and/or increase of the amount of product of the reaction catalyzed by cardiac ADPRC.

Evidently, the enzyme activity is influenced by a series of factors including the amount of enzyme, the activation status of the enzyme, the presence of cofactors such as a co-activator or a co-repressor, the presence of activators and inhibitors and the ambient condition such as salt concentration, temperature, pH etc. Usually, the enzyme activity is measured at standard laboratory conditions and may be adapted to the optimum of the test system in question. Accordingly, the test system may be used in order to detect or identify molecules changing the activation status of the enzyme such as activators and inhibitors, which might be useful therapeutics.

In order to obtain the desired enzyme activity, the measurements may be carried out in a molecular environment suitable for the activity of the cardiac ADPRC enzyme. Preferably, the method is carried out in a aqueous buffer of neutral pH, more preferably in a buffer with a pH around pH 7, even more preferably in a buffer of pH 7 comprising Tris-Malat, potassium chloride (KCl), sucrose, complete protease inhibitors and/or dimethyl sulphoxide (DMSO). Preferably, the cardiac ADPRC maintains its activity in the used buffer and preferably compound or enzyme precipitation is prohibited. Optionally, the samples may be incubated at a temperature suitable for the enzyme activity, preferably at temperatures between 0 and 50°C, more preferably at temperatures between 10 and 40 °C, even more preferably at temperatures between 20 and 40 °C, most preferably at 37°C. The applied methods are described in more detail in the method section. However, it should be understood that these are exemplary and other methods may be appropriate as well.

It might be obvious to a person skilled in the art that the enzyme has to be diluted to the desired concentration and that this concentration may vary for different batches due to the varying enzymatic activity per amount of enzyme.

As mentioned above, the cardiac ADPRC used for the assay may be membrane-bound, may be immobilized on surfaces (such as microarrays, beads and the like) or may be dissolved in a buffer. Preferably, the cardiac ADPRC is membrane-bound, more preferably the cardiac ADPRC is bound to a sarcoplasmatic reticulum membrane.

In the present test system, detectable surrogates of the substrate adenosine diphosphoribose (NAD) may be used to enable the detection of the enzymatic activity of the cardiac ADPRC. Preferably, these detectable surrogates may be detectable by physicochemical means, more preferred by optical means such as fluorescence detection or measurement of the absorbance.

Even more preferred, the molecules detectable by optical methods may be (i) molecules that alter their fluorescence spectrum upon conversion by the enzyme, (ii) non-fluorescent precursors that form fluorescent products of fluorescent agents upon conversion by the enzyme, (iii) surrogates or molecular complexes detectable by their photochemical properties (e.g. their characteristic absorbance in the UV, the IR or the visible spectrum or by their fluorescence), (iv) surrogates that may be cleaved into products with altered absorbance spectrum (e.g. p-nitrophenyl esters) upon conversion by the enzyme, (v) precursors that emit photoluminescence upon conversion by the enzyme or (iv) molecules labeled with one or more dyes (e.g. a dye detectable by UV/Vis spectrophotometry, a fluorescent dye such as a Cy dye (e.g. Cy3, Cy5, Cy5), an Alexa dye (e.g. Alexa488, Alexa 546, Alexa 647), a S dye (e.g. S0387), fluorescein and functional derivatives thereof (e.g. FITC), rhodamine, HOECHST dye).

Preferably, the detectable surrogates may be molecules that alter their fluorescence spectrum or intensity upon conversion by the enzyme, non-fluorescent precursors that form fluorescent products of fluorescent agents upon conversion by the enzyme, or surrogates that may be cleaved into products with an altered absorbance spectrum or altered absorbance intensity, more preferably molecules that alter their fluorescence spectrum or intensity upon conversion by the enzyme, non-fluorescent precursors that form fluorescent products of fluorescent agents upon conversion by the enzyme, even more preferably nicotine purine nucleotide derivatives, most preferably nicotinamide adenine dinucleotide (NAD), nicotinamide guanidine dinucleotide (NGD) or nicotinamide hypoxanthine dinucleotide (NHD). The NAD, NGD and/or NHD is/are converted to cyclic adenosine diphosphoribose (cADPR), cyclic guanosin diphosphoribose (cGDPR) and cyclic inosine diphosphoribose (cIDPR), respectively, that can be detected, e.g., via their fluorescence. Alternatively the detectable surrogates may also be molecules labeled with a binding partner of high-affinity binding (such binding partners are, e.g., biotin-streptavidin, methotrexate-dihydropholate, etc.) or radioactively labeled surrogates.

The enzymatic activity of the cardiac ADPRC may be measured using above-mentioned surrogates, in particular by measuring alterations of a signal occurring from the detectable molecule over time, in more particular, alterations of a fluorescent signal over time. Preferably, the fluorescent signal over time is measured after certain time intervals, more preferably these time intervals are in the range of several minutes. Between the measurements, the samples may be incubated at the desired conditions.

In preferred embodiment, the activity of the cardiac ADPRC is determined by measuring the conversion of a substrate of the cardiac ADPRC into a product. The conversion of a substrate relates to the enzymatic, thus, to the cardiac ADPRC-catalyzed alteration or metabolism of a chemical compound. Throughout the invention, the term "substrate" relates to any chemical substance that may be metabolized by cardiac ADPRC. Preferably, the substrate is a small molecule of a molecular weight of less than 500 daltons.

In a preferred embodiment, the substrate is nicotinamide adenine dinucleotide (NAD), nicotinamide guanidine dinucleotide (NGD) or nicotinamide hypoxanthine dinucleotide (NHD). Thereby, the conversion of a substrate is preferably measured by a method that can be read out effectively, e.g., via an enzymatic assay, preferably read out via fluorescence detection. The substrate, in particular a surrogate of NAD, may be added (i) to the subject the biological material is obtained from, thus, to the animal excluding human, to the tissue or to the cell(s), (ii) to the reaction mixture before the experiment begins, (iii) during the incubation of the sample, (iv) after the incubation of the sample but before the measurement or (v) after the incubation of the sample and during the measurement.

Additionally, one or more compounds of interest may be added to the reaction mixture. The compound(s) may be added (i) to the subject the biological material is obtained from, thus, to the animal excluding human, to the tissue or to the cell(s), (ii) to the reaction mixture before the experiment begins, (iii) during the incubation of the sample, (iv) after the incubation of he sample but before the measurement or (v) after the incubation of he sample and during the measurement.

Preferably, the enzymatic activity of the cardiac ADPRC is determined by an enzymatic assay. More preferably, the activity of the cardiac ADPRC is determined by fluorescence.

The term "fluorescence" is used herein as known in the art. Preferably, the term "fluorescence" refers to the emission of light of a longer wavelength by a molecule or a group of molecules that have been excited by light of a shorter wavelength. Alternatively, the term fluorescence may also include two-photon effects, thus the emission of one photon by a molecule excited with two photons of a longer wavelength. Furthermore, other photophysical effects such as effects dependent on triplet states such as phosphorescence may be included.

As described above, the term "determined by fluorescence" preferably refers to the detection of the alterations of the fluorescent signal over time may be measured by (i) detecting alterations of the fluorescence intensity in a fluorescence spectrophotometer or in a microscopic device, (ii) detecting alterations of the fluorescence spectrum in a fluorescence spectrophotometer or in a microscopic device, (iii) detecting alterations of the mobility of the fluorescent molecule in a suitable device such as via a microscopic device, via fluorescence correlation spectroscopy (FCS), via fluorescence cross-correlation spectroscopy (FCCS) or via a fluorescence depolarization assay, via fluorescence resonance energy transfer (FRET)-based methods or via an amplified luminescence proximity homogeneous assay (ALPHA). These detection techniques may be known to a person skilled in the art. Preferably, the detection of the alterations of the fluorescent signal over time may be measured by detecting alterations of the fluorescence intensity. More preferably, the signal is measures in a fluorescence spectrophotometer, even more preferably in a fluorescence spectrophotometer suitable for microtiter plates. Preferably, the fluorescence is detected by excitation in the UV range and the emitted light is detected at a longer wavelength, more preferably the excitation is between 250 and 300 nm and the detection is between 380 and 450 nm, even more preferably the excitation is at 275 nm and the detection is between 400 and 420 nm, most preferably the excitation is at 275 nm and the detection is at 410 nm. The applied methods are described in more detail in the method section. However, it should be understood that these are exemplary and other methods may be appropriate as well. The skilled person might be able to adjust the excitation and detection conditions to the molecules and the measurement method in use.

The cardiac ADPRC activity of each sample may be defined by the change in fluorescence with time, i.e., the slope of the line in the fluorescence versus time. These results may be obtained from a linear regression as shown in the examples. The addition of a compound that bears an inhibitory effect will lead to a slower conversion of the observed substrate and a slower generation of product and therefore a flatter kinetic curve. The addition of a compound that bears an activating effect will lead to a faster conversion of the observed substrate and a faster generation of product and therefore a steeper kinetic curve. The addition of a compound that bears no effect will not influence the enzymatic conversion of the substrate and the generation of the product and therefore does not change the steepness the kinetic curve.

Accordingly, concentration-response curves may be obtained from these results. These results may be displayed as a dose-response curve and fitted with different models, in particular with models used in pharmacologic studies, in more particular by a Hill equation well-known to a person skilled in the art. Finally, half-maximal effective concentrations ($EC_{50}$ values) and/or half-maximal inhibitory concentrations ($IC_{50}$ values) may be calculated. Preferably, $IC_{50}$ values are calculated.

If the respected compound shows activity as inhibitor or activator this may result in an alteration in the enzymatic activity of cardiac ADPRC. The potency of the compound may be obtained by measuring the alteration in the enzymatic activity. If the tested compound has a specific and significant effect on the test system, the compound is identified as a modulator of cardiac ADPRC. In the context of the present invention, the compound has an antiarrhythmic effect. Therefore, it is preferably an inhibitor of cardiac ADPRC. The result of several measurements may be compared and analyzed by mathematical means such as statistical means, e.g., a Student's t-test or a Chi-square test.

Fluorescence polarization (FP)-based assays are assays which use polarized light to excite fluorescent substrate or compound in solution. These substrates or compounds are free in solution and tumble, causing the emitted light to become depolarized. When the substrate compound binds to a larger molecule or its size is significantly decreased by cleavage, its tumbling rates are greatly decreased, and the emitted light remains highly polarized.

Fluorescence correlation spectroscopy (FCS) is a widely used method based on a confocal optical system that measures the velocity of fluorescent compounds in solution. As compound bound to high-molecular targets are slower due to their larger Stokes radius the binding of fluorescent molecules and/or their cleavage may be detected. Fluorescence cross-correlation spectroscopy (FCCS) is a method similar to FCS but detecting two or more different fluorophores. It provides all results obtained by FCS and additionally gives information regarding the ratio of two differently labeled fluorescent particles diffusing conjointly. Consequently, FCCS provides information on the binding of two differently labeled binding partners or on cleavage of a compound between two different binding fluorophores.

ALPHA is a solution-based assay which was originally developed by Packard BioScience. ALPHA is a luminescence-based proximity assay, wherein one interaction partner is attached to donor beads, while the other is coupled to acceptor beads, both with a diameter of only about 250 nm. A photosensitizer compound is embedded into the donor bead. With this compound upon illumination with laser light at a wavelength of about 680 nm, ambient oxygen is converted into energy-rich, short-life singlet oxygen. When no acceptor bead is in proximity, the singlet oxygen decays without producing a signal. If donor and acceptor bead are brought together (ca. 250 nm) by the biological interaction of the attached biomolecules, the singlet oxygen released by the donor bead initiates a luminescence/fluorescence cascade in the nearby acceptor bead, leading to a highly amplified signal in the 520 nm to 620 nm range. The luminescence signal is detected in a suitable reader. For more details regarding ALPHA techniques, see Ullman et al., 1994, Proc. Natl. Acad. Sci., USA 91, 5426-5430.

Alternatively, the enzyme activity may also be measured by immunochemical methods including but not limited to enzyme linked immuno sorbent assay (ELISA) including sandwitch ELISA. Here, the substrate of the conversion product of the enzymatic reaction may serve as an antigen or a haptene as known in the art. Converted and non-converted samples may provoke different signal intensities. ELISA-based assays are offered by various companies. It is a biochemical technique used to detect the presence of an antibody or an antigen in a sample. The antigen may also be a small molecule, such as a substrate or a conjugate of a substrate of cardiac ADPRC or a conversion product of the reaction catalyzed by the cardiac ADPRC. Furthermore, the substrate or conversion product may also serve as haptene. Performing an ELISA involves at least one antibody with specificity for a particular. In general, an unknown amount of antigen in a sample is immobilized on a surface. A particular antibody is washed over the surface. This antibody is linked to an enzyme that produces a detectable signal such as a change in color or fluorescence. For example, the sample with an unknown amount of antigen is immobilized on a solid support (usually a microtiter plate) either non-specifically (via adsorption to the surface) or specifically (via capture by another antibody specific to the same antigen, in a "sandwich" ELISA). After the antigen is immobilized the detection antibody is added, forming a complex with the antigen. The detection antibody can be covalently linked to an enzyme, or can itself be detected by a secondary antibody which is linked to an enzyme through bioconjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. Older ELISAs utilize chromogenic substrates, though newer assays employ fluorogenic substrates with much higher sensitivity.

As a further alternative, the enzyme activity may also be measured by techniques based the measurement of alterations in the retention time in chromatographic methods (e.g. affinity chromatography, ion exchange chromatography, size-exclusion chromatography, HPLC, FPLC and/or UPLC). In particular, if the retention times of the substrate and the conversion product are distinguishable or the retention times of the unbound substrate and the cardiac ADPRC are distinguishable chromatographic methods may be preferably used.

In a preferred embodiment, the method is used to screen a library of compounds. In this context, the library of compounds may be composed of a plurality of chemical substances that may be assembled of multiple sources. A library may include chemically synthesized substances, products of biotechnological processes, naturally occurring substances or products resulting from combinatorial chemistry techniques or combinations thereof. In the context of the present invention, the library preferably comprises small molecule compounds, more preferably chemically synthesized small molecule compounds.

In the context of the present invention the term "screen" relates to a method in which a standardized molecular assay or a composition of several molecular assays is applied to a plurality of compounds to determine their properties of interest such as the binding to molecular targets or the biological activity, in particular the influence on the enzymatic activity, in more particular the inhibition of the enzymatic activity. A screen may be carried out in solution, e.g., in flasks, reaction tubes, cuvettes, microtiter plates and the like, at a surface, e.g., in a microarray format or on a bead based format, in a living animal excluding human or in a living cell. Preferably, the screen may be carried out in solution. The screen may preferably be carried out with little compound consumption and/or small volumes. Therefore the use of a microtiter format is preferred. On such a microtiter plate, only small amounts such as few microliters may be sufficient for the screen.

In a preferred embodiment, the method is carried out in an automated format, particularly in a high throughput format. In the context of the present invention, the term "automated format" refers to a method that is fully or partly controlled and/or carried out by one or more technical devices, preferably pipetting robots. In this context, the term "high throughput format" relates to an assay system for the rapid testing of a plurality of compounds within in a short time, thus, the assaying time per tested compound is minimized. The assay is preferably carried out in multiwell plates, more preferably in 6-well plates, 12-well plates, 24-well plates, 96-well plates or 384-well plates, even more preferably in 96-well plates or 384-well plates.

A second aspect of the invention relates to the use of cardiac ADPRC for the identification of a compound having an antiarrhythmic effect.

In a preferred embodiment, the cardiac ADPRC is an intracellular membrane-bound cardiac ADPRC, particularly a sarcoplasmatic reticulum membrane-bound cardiac ADPRC or as cardiac ADPRC of mammalian origin, in particular from human, pig, guinea pig or rat, especially pig or human heart.

In order to confirm the antiarrhythmic effect determined by the method described above, the cardiac ADPRC activity or the antiarrhythmic properties of cardiac ADPRC inhibitors may be determined in a cell, a tissue or in an organ. Preferably, wherein the cell or tissue from heart or the organ is a heart, more preferably a muscle cell or muscle tissue from heart, even more preferably a papillary muscle cell or papillary muscle tissue, most preferably a papillary muscle tissue. The cell or the tissue may be either freshly prepared from animals excluding human or be obtained from cell culture and/or tissue culture techniques as described above. In another preferred embodiment, a muscle is used for the measurements, preferably a heart muscle, even more preferably a papillary muscle. In a further preferred embodiment, a freshly prepared or extracted heart is used.

Preferably, the action potential is measured in an organ, in particular in a heart, in a tissue, in particular in a heart muscle tissue or in a cell, in particular a heart muscle cell. In this context, the action potential may be understood as the ion in- and efflux into a cell, a tissue or an organ such as a papillary muscle, the resulting electrical voltage alteration and/or the scalar potential of the heart, often measured as electrocardiography (ECG). Preferably, the signal is accompanied by alternating calcium in- and efflux and/or alternating electric voltage at the plasma membrane of a cell or a tissue. In the present case, the action potential may be preferably determined using electrical detection methods, more preferably methods measuring the electrical field or alternations thereof, even more preferably electrophysiological recordings. The effect of the compounds that might be tested on the action potential of a mounted papillary muscle, a papillary muscle cell or a papillary muscle tissue may be tested. Therefore the respective samples may be preincubated with the compound or incubated without compound, the compound may be added before the experimental proceedings are conducted, during the experimental proceedings of the method or at the end of the experimental proceedings.

In a preferred embodiment, the contraction of the papillary muscle, the papillary muscle cell or the papillary muscle tissue is measured. For this purpose, the papillary muscle or a cell or tissue thereof may be mounted in a device suitable to detect the contraction. The mounted papillary muscle, the papillary muscle cell or the papillary muscle tissue may then be stimulated with electric pulses. Preferably, the electric pulses are rectangular pulses of few volts, more preferably rectangular pulses of 0 to 20 volts, even more preferably rectangular pulses of 0 to 10 volts, most preferably rectangular pulses of 1 to 4 volts. The duration of the pulses may be few milliseconds (ms), preferably the duration of the pulses may be 0 to 20 ms, more preferably the duration of the pulses may be 0 to 10 ms, even more preferably the duration of the pulses may be 1 to 3 ms. The sample may be paced at several Hz for few seconds, preferably at 0 to 10 Hz for 0 to 60 s, more preferably at 0 to 10 Hz for 30 s and even more preferably at 4 Hz for 30 s. During the experimental proceedings, the observed muscle, tissue or cell may be in a buffer solution and/or perfused by a buffer solution. The buffer solution may be suitable for heart cell activity measurement. Preferably, the buffer is either a commercially available buffer or a buffer containing calcium chloride ($CaCl_2$). The concentration of $CaCl_2$ may vary and may be increased up to 5.5 mM or even higher. Preferably the buffer has a temperature suitable for heart cell, tissue or muscle activity, more preferably a temperature below 40°C, even more preferably a temperature of 10 to 40°C, most preferably a temperature of ca. 37°C. Action potentials may be recorded at different times. They may be recorded directly, after several seconds and/or after several minutes.

In the above-mentioned arrangement, the action potentials may be recorded by means of any device known in the art to detect action potentials, such as molecular imaging methods or electrophysiological methods. Preferably electrophysiological methods may be used, more preferably the detection of the action potentials is performed via an electrode. The electrode may be of any conductive material such as metal or of a nonconductive material filled with a conductive solution. Preferably, the electrode is a glass electrode filled with an aqueous buffer, more preferably the electrode is a glass electrode filled with an aqueous potassium chloride (KCl) solution, even more preferably with a 3 M KCl solution. The electric signal occurring from the action potentials may be amplified if required and may be detected by and/or stored in a computer based system. Preferably, the measured data are analyzed by a computer-based system.

Alternatively, or additionally the action potential may be detected indirectly via a molecular imaging method and/or flow cytometry. In this context, e.g., voltage sensitive dyes may be used or the calcium influx and/or efflux may be recorded by calcium sensitive dyes. These dyes are well-known to a person skilled in the art. The detected signal, such as images, signals or plots, in particular fluorescence images or signals, occurring from the action potential may be amplified if required and may be detected by and/or stored in a computer based system. The cells may be grown in multichamber plates or on microarray slides to enable higher throughput of the number of analyzes cells and/or automated microscopy. Preferably, the obtained microscopic images are analyzed by automated or semiautomatic image processing in order to quantify and compare the cellular brightness and/or the brightness in certain cell compartments.

In the case that the tested compound has a specific and significant effect on the test system, the compound is identified as a modulator of cardiac ADPRC. In the context of the present invention, the compound has an antiarrhythmic effect. Therefore, it is preferably an inhibitor of cardiac ADPRC. The result of several measurements may be compared and analyzed by mathematical means such as statistical means, e.g., a Student's t-test or a Chi-square test.

Beside the use of organs, tissues and cells, the antiarrhythmic effect may be detectable in *in vitro* studies based on living systems that are considered to be *in vitro* and *ex vivo,* respectively. In these studies the contraction of the observed cells, tissues or organs is observed visually, by automated image processing or by measurement of the physical force resulting from the contraction or by electrophysiological recordings. The term "living systems that are considered to be *in vitro* and *ex vivo*" may refer to extracted organs such as heart or frog's leg widely used as model systems to determine action potentials and/or contractility of muscles or it may refer to egg-based systems, in particular *in ovo* studies, in more particular based on *in ovo* studies in hen's egg widely used in pharmaceutical research. Alternatively or additionally, the antiarrhythmic effect may be detectable in *in vivo* studies using animals excluding human. The compound may be administered into the animals by any means, including but not limited to injection, inhalation, and administration via the skin or the mucosa and by oral uptake. In the context of the present invention, the method in an animal model may not be considered as a method for treatment or as a diagnostic method, but merely serves as a detection method to identify one or more antiarrhythmic effects of the compound or the compounds tested. This may not exclude the potential usefulness of certain compounds inhibiting or enhancing the activity of cardiac ADPRC in therapeutic and/or diagnostic methods. The heart beat and/or the electrocardiography (ECG) signal of the animals may be recorded and analyzed. Alternatively, the conversion of a substrate may be measured systemically, thus, the substrate and/or its metabolic substrate may be detected in blood or other body liquids or the conversion is detected *in situ,* e.g., by *in vivo* imaging techniques such as *in vivo* fluorescence detection as known in the art.

The invention is further explained by the following examples and figures, which are intended to illustrate, but not limit the scope of the present invention.

FIGURES

Figure 1 shows the time dependence of the conversion of NHD to clDPR catalyzed by pig cardiac sarcoplasmatic reticulum membranes. clDPR formation was monitored by following the change in fluorescence at excitation and emission wavelengths of 275 and 410 nm, respectively. Data is shown for the positive control ("high"), the back ground sample ("low") and a test compound ("cpd") giving 47 % inhibition. The slope of the line fitted to the points is taken as the measure of enzymatic activity.

Figure 2 illustrates the structure of A001102589, identified as an inhibitor of cardiac ADPRC.

Figure 3 shows the concentration response curve for the inhibitor compounds A001102589 and SW64825. The $IC_{50}$ values were determined to be 0.21 and 0.04 $\mu$M, respectively.

Figure 4 demonstrates that the cardiac ADPRC inhibitor A001102589 significantly reduced the number of delayed after-depolarizations observed within 6 s after high-frequency pacing of guinea pig papillary muscle.

Figure 5 demonstrates a further cardiac ADPRC inhibitor from a different structural class than A001102589 (A), SW64825 (B), that reduce the number of delayed after-depolarisations observed within 6 s after high-frequency pacing of guinea pig papillary muscle. The concentration of the compound was 3 $\mu$M and the DMSO sample represents 0.03 % DMSO. The compound SW64825 reduces the number of delayed after-polarisations significantly.

Figure 6 shows the influence of the test compound SW64825 on the time to cardiac arrest (A) and the time to ventricular fibrillation (B) after ouabain infusion and an intravenous (i.v.) infusion of each 3 mg/kg of the compound. The control indicates an animal without treatment with the compound.

Figure 7 illustrates the structure of SW64825, identified as an inhibitor of cardiac ADPRC.

EXAMPLES

Example 1: Measurement of nucleoside diphosphoribosyl cyclase activity

Experimental protocol. Sarcoplasmatic reticulum membranes from pig heart were prepared as described by Kranias et al. (Biochim. Biophys. Acta 709, 28-37, 1982), with slight modifications. The left ventricle of a freshly explanted piglet heart was cleaned of fat and connective tissue and cut into small pieces (approximately 1 cm$^3$). The pieces were briefly rinsed with ice-cold medium I (30 mM Tris-Malat, pH 7.0, 0.3 M Sucrose, 5 mg/ml Leupeptin, 0.1 mM PMSF) and homogenised in ice-cold medium I (5 ml/g tissue) using a Waring blender (60 s at full speed, divided into four 15 s periods with 30 s cooling intervals in-between). The homogenate was the centrifuged for 10 min at 5,500 g. The supernatant was passed through four layers of Miracloth (Merck Biosciences, Darmstadt, Germany) and centrifuged at 12,000 g for 25 min. The supernatant was again filtered through four layers of Miracloth and then centrifuged at 143,000 g for 60 min. The resulting pellet was re-suspended in medium II (20 mM Tris-Malat, pH 7.0, 0.6 M KCl, 0.3 M Sucrose, 5 mg/ml Leupeptin, 0.1 mM PMSF) using an Ultra-Turrax device, and the suspension was again centrifuged at 143,000 g for 60 min. The pellet was re-suspended in medium I and again centrifuged at 143,000 g for 60 min. The pellet from this last centrifugation step was resuspended in medium III (20 mM Tris-Malat, pH 7.0, 0.1 M KCl, 0.3 M Sucrose, Complete protease inhibitors (Roche, Penzberg, Germany) w/o EDTA) to give a final protein concentration between 5 and 10 mg/ml. The suspension was aliquoted, shock-frozen in liquid nitrogen and stored at -80 °C.

Nicotinamide guanidine dinucleotide (NGD) and Nicotinamide hypoxanthine dinucleotide (NHD) were taken as surrogates for nicotinamide adenine dinucleotid (NAD) to measure cardiac ADPRC activity as described by Graeff et al. (Biochemistry 35, 379-386, 1996). In the reaction catalyzed by the cyclase, NGD and NHD are converted to cyclic guanosine diphosphoribose (cGDPR) and cyclic inosine diphosphoribose (cIDPR), respectively, that can be detected via their fluorescence in the visible spectrum. The method was carried out as follows:

Test compounds (1 $\mu$l stock solution, 3 mM in DMSO) were diluted in 99 $\mu$l reaction buffer (20 mM Tris-Malat, pH 7.0, 0.1 M KCl, 0.3 M Sucrose, Complete protease inhibitors w/o EDTA) containing 2 % DMSO to yield 30 $\mu$M test compound in reaction buffer containing 3 % DMSO. Cardiac sarcoplasmatic reticulum membranes prepared as described above were diluted in reaction buffer to a concentration giving sufficient substrate turnover within the observation time. The appropriate dilution needs to be determined individually for each batch of cardiac SR membranes. Subsequently, the substrate (NGD or NHD, 10 mM stock solution in water) was diluted to a concentration of 750 $\mu$M in reaction buffer. 2 $\mu$l of compound solution were transferred to a 384-well small volume microtiter plate (Greiner Bio-One, Frickenhausen, Germany). 2 $\mu$l of diluted SR membranes were added, and the reaction was started by addition of 2 $\mu$l substrate solution. Positive controls (reaction buffer with 3 % DMSO instead of compound solution) and background samples (reaction buffer with 3 % DMSO instead of compound solution, reaction buffer instead of cardiac SR membranes in reaction buffer) were included on each plate. Then, the fluorescence of the reaction mixture (excitation wavelength 275 nm, emission wavelength 410 nm) was measured directly after addition of substrate (to measurement) and after several incubation periods (e.g., 30, 60, 90 min). Between measurements, the plate was incubated at 37 °C.

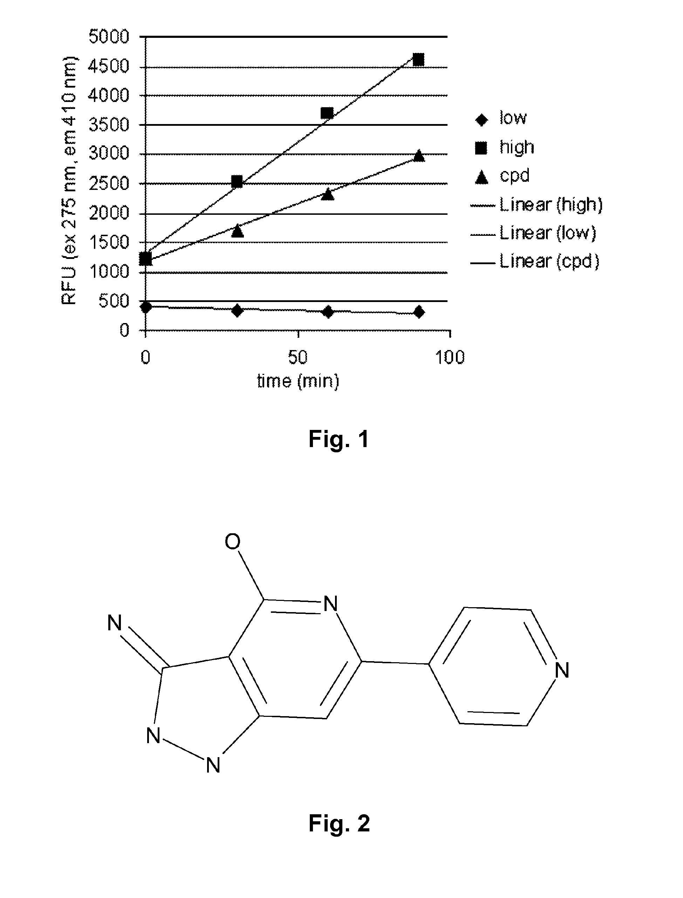

Analysis. The cyclase activity of each reaction mixture was defined by the change in fluorescence with time, i.e., the slope of the line in the fluorescence vs. time plot obtained by linear regression as shown in figure 1. Percent-Inhibition values (1%) were obtained by using the following equation:

$$I\% = 100 \cdot \left( 1 - \frac{slope(sample) - slope(negative\_ctrl)}{slope(positive\_ctrl) - slope(negative\_ctrl)} \right)$$

Concentration-response curves were obtained by testing compounds over a range of different concentrations. The $IC_{50}$ value was used as the measure of potency of test compounds, it was calculated by fitting the data points to the following equation (parameter C). Parameter A is the lower limit for I% at low compound concentration. It was set to zero. Parameter B is the upper limit for I% at high compound concentration. Parameter D is the so-called Hill coefficient, it defines the slope of the concentration response curve at its inflection point.

$$I\% = A + \frac{B - A}{1 + \left( \dfrac{C}{[Compound]} \right)^{D}}$$

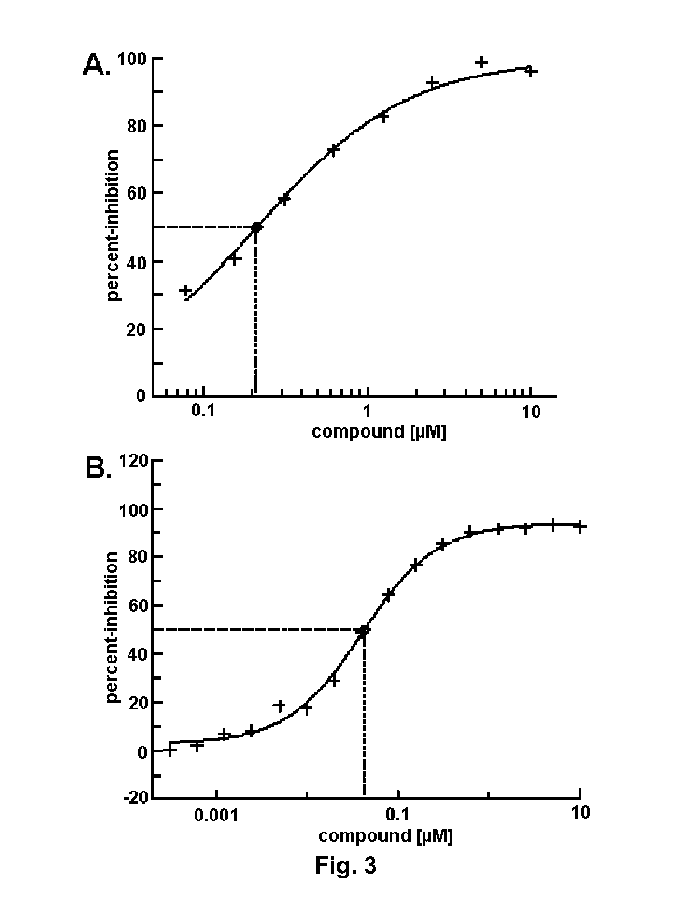

Results. A library of approximately 16,000 compounds was screened and several cardiac ADPRC inhibitors were identified, that showed a significant decrease in the steepness of the kinetic curve of the substrate conversion catalysed by the cardiac ADPRC (example shown in figure 1). Exemplarily, the results for 2 different cardiac ADPRC inhibitors are shown here. The enzyme kinetic values are depicted in a dose-response curve (figure 3) from which the $IC_{50}$ value was deduced. The $IC_{50}$ value for compound A001102589 was approximately 0.21 $\mu$M. The $IC_{50}$ value of the compound SW64825 was approximately 0.04 $\mu$M. Surprisingly, these results correlate very well with the anti-arrhythmic properties of the compounds as described below.

Example 2: Determination of antiarrhythmic properties of cardiac ADPRC inhibitors *in vitro*

Antiarrhythmic properties of cardiac ADPRC inhibitors identified with the assay described above were probed for by determining their ability to prevent the occurrence of delayed after-depolarizations in guinea pig papillary muscle cells following high-frequency electrical stimulation. The experimental protocol was as follows:

Tissue preparation. Guinea pigs (Dunkin Hardley Pirbright), weighing approximately 400 g, were sacrificed by concussion followed by exsanguination. Immediately after opening of the thorax the heart was removed and was immersed in Tyrode solution (in mmol/l): 136 NaCl, 3.3 KCl, 1.2 $KH_2PO_4$, 1.1 $MgSO_4$, 2.5 $CaCl_2$, 5 Glucose, 10 HEPES, and pH= 7.4, gassed with oxygen), at room temperature. Finally, the left papillary muscle was removed and mounted in a measuring chamber, containing Tyrode solution heated to 37°C. The time from removal of the heart until mounting the papillary muscle in the chamber was approximately 3 min.

Electrophysiological recordings. Immediately after mounting of the papillary muscle, it was stimulated with rectangular pulses of 1 to 4 Volts and duration of 1 to 3 ms, at a frequency of 1 Hz, by means of a computer programme (MFK, Niedernhausen, Germany). The bath was continuously perfused with Tyrode solution by means of a roller pump (TL, Meredos GmbH, Bovenden, Germany) at a rate of 4 ml per min. The solution was preheated to 37°C. Action potentials (APs) were recorded by means of a glass microelectrode, filled with 3 M KCl solution. The electrodes were fabricated from borosilicate glass (item number: 1 B150F-4, World Precision Instruments, Sarasota, USA), by means of a microelectrode puller (DMZ-Universal Puller, Zeitz Instruments, Martinsried, Germany). The electrodes had an electrical resistance of 5 to 10 megaohms. The electrical signal was recorded with an amplifier (Model 309, Harvard Apparatus GmbH, March-Hugstetten, Germany), and stored in a computer system.

Experimental protocol. After an equilibration period of 30 min, an AP was recorded and the compound or control is added to the perfusion solution. 15 min later, an AP was recorded and the perfusion solution is modified: KCl and $KH_2PO_4$ were omitted and $CaCl_2$ was increased to 5.5 mM. After 15 min, an AP was recorded and the tissue was paced at 4 Hz for 30 s. Then pacing was stopped and frequently spontaneous APs occur. The number of spontaneous APs occurring 6 s after cessation of pacing was counted. In the presence of the background sample only, the number of spontaneous APs in the 6 s period was approximately 13. The number of measurements with control and compound was 10, for each. The numbers of spontaneous APs in each group were compared by means of a Student's t-test.

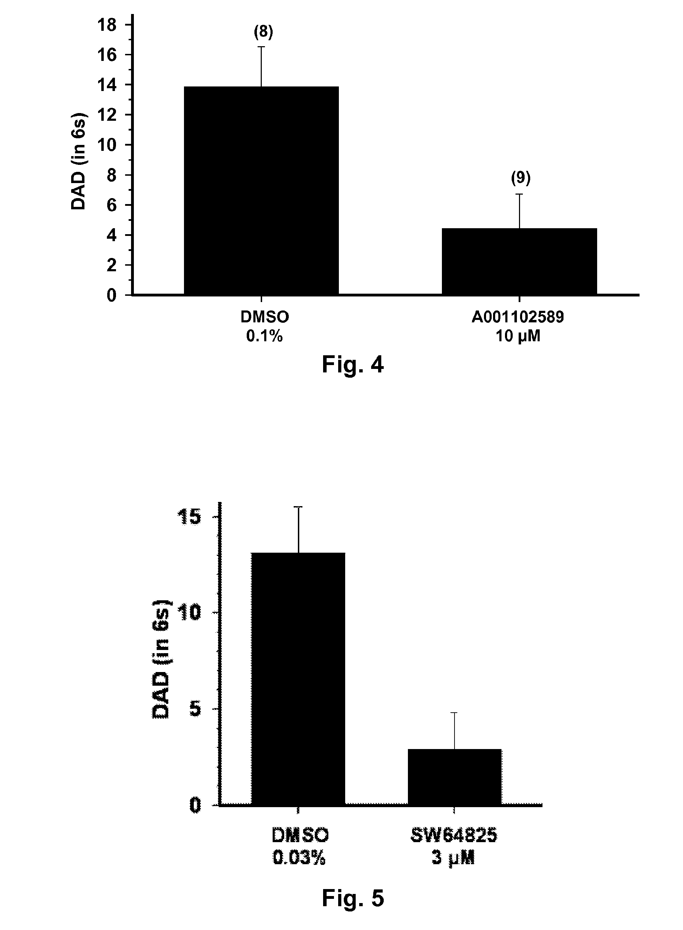

Results. The results of the depolarization experiments are summarized in figure 4 and 5. At 10 $\mu$M, the cardiac ADPRC inhibitor A001102589 significantly reduced the number of delayed after-depolarizations within 6 s following rapid pacing to averagely 4.4 depolarisations in 6 s, whereas the DMSO control shows approximately 14 depolarizations in 6 s (figure 4). In another experiment, the effect of the cardiac ADPRC inhibitor SW64825 was assayed. At 3 $\mu$M, the cardiac ADPRC inhibitor SW64825 significantly reduced the number of delayed after-depolarisations within 6 s following rapid pacing. Whereas in the DMSO control there were averagely 13.1 depolarisations in 6 s, the powerful cardiac ADPRC inhibitor SW64825 reduced the number of depolarisations to averagely 2.9 depolarisations in 6 s.

Surprisingly, these results overall correlate very well with the results found for the inhibition of the enzymatic activity of cardiac ADPRC as shown above.

Example 3: Determination of anti-arrhythmic properties of cardiac ADPR cyclase inhibitors *in vivo*

Experimental protocol. Antiarrhythmic properties of ADPR cyclase inhibitors were measured *in vivo* in anesthetized guinea pigs. For this purpose, guinea pigs are anesthetized with pentobarbital (100 mg/kg i.p.) and are ventilated with 40 % (v/v) oxygen ($O_2$). After 30 min the compound is applied as bolus injection. 5 min later, ouabain is infused at a rate of 30 pg/kg per min, causing a strong continuous increase in contractility. In the presence of the background sample, ventricular fibrillation arises approximately 12 min after infusion of ouabain and cardiac arrest occurs approximately after 15 min.

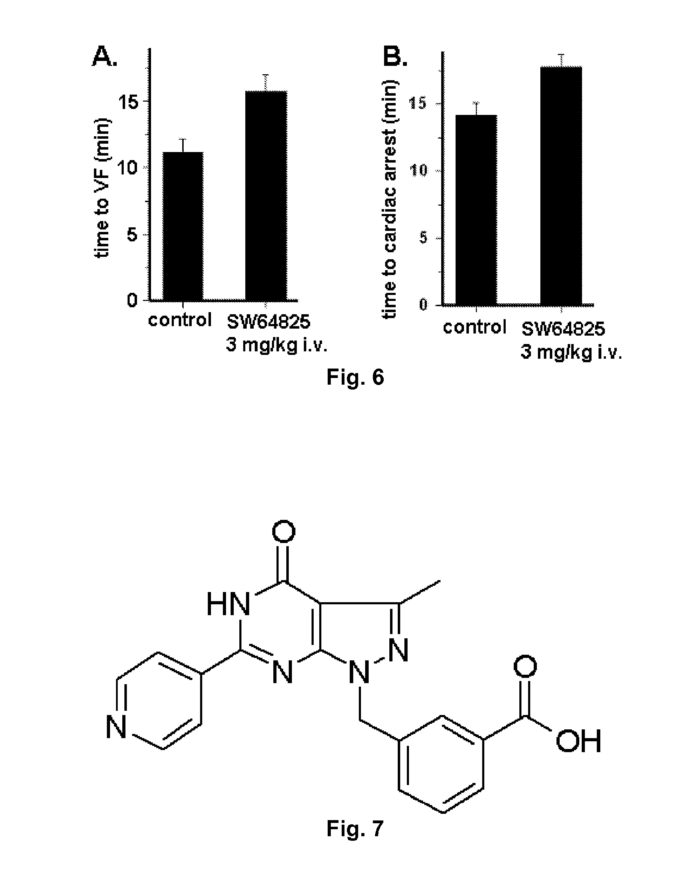

Results. In the presence of a cardiac ADPRC inhibitor, time to ventricular fibrillation (VF) and cardiac arrest were significantly prolonged. The results are illustrated in figure 6. The cardiac ADPRC inhibitor SW64825 significantly prolonged the time to ventricular fibrillation and cardiac arrest upon ouabain infusion into anesthetized guinea pig.

REFERENCES

1. Graeff et al., Biochemistry 35, 379-386, 1996
2. Kranias et al., Biochim. Biophys. Acta 709, 28-37, 1982
3. WO 08/82169, Chonbuk University, S. Korea

4. Ullman et al., Proc. Natl. Acad. Sci., USA 91, 5426-5430, 199

**Claims**

1. A method of identifying a compound having an antiarrhythmic effect, the method comprising

   - providing a cardiac adenosine-diphospho-ribose cyclase (cardiac ADPRC),
   - contacting the cardiac ADPRC with a compound, and
   - determining the activity of the cardiac ADPRC in the presence of the compound,

   wherein a reduced activity of the ADPCR relative to a control is indicative of the antiarrhythmic effect of the compound.

2. The method of claim 1, wherein the cardiac ADPRC is an intracellular membrane-bound cardiac ADPRC, particularly a sarcoplasmatic reticulum membrane-bound cardiac ADPRC.

3. The method of claim 1 or 2, wherein the cardiac ADPRC is of mammalian origin, in particular from human, pig or rat, especially pig or human heart.

4. The method of any of claims 1 to 3, wherein the activity of the cardiac ADPRC is determined by measuring the conversion of a substrate of the cardiac ADPRC into a product.

5. The method of claim 4, wherein the substrate is nicotinamide adenine dinucleotide (NAD), nicotinamide guanidine dinucleotide (NGD) or nicotinamide hypoxanthine dinucleotide (NHD).

6. The method of any of claims 1 to 5, wherein the activity of the cardiac ADPRC is determined by fluorescence.

7. The method of any of claims 1 to 6, wherein the method is used to screen a library of compounds.

8. The method of any of claims 1 to 7, wherein the method is carried out in an automated format, particularly in a high throughput format.

9. Use of cardiac ADPRC for the identification of a compound having an antiarrhythmic effect.

10. The use of claim 9, wherein the cardiac ADPRC is further defined as in claim 2 or 3.

Fig. 1

Fig. 2

Fig. 3

**Fig. 4**

**Fig. 5**

Fig. 6

Fig. 7

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/40799 A2 (CV THERAPEUTICS INC [US]; BELARDINELLI LUIZ [US]; GAO ZHENHAI [US]) 7 June 2001 (2001-06-07) * p. 2, line 17 - 25, p. 5, line 21 - p. 6, line 6 * ----- | 1-10 | INV. G01N33/50 |
| A | GUL RUKHSANA ET AL: "Inhibition of ADP-ribosyl cyclase attenuates angiotensin II-induced cardiac hypertrophy." CARDIOVASCULAR RESEARCH 15 FEB 2009 LNKD-PUBMED:18719074, vol. 81, no. 3, 15 February 2009 (2009-02-15), pages 582-591, XP002599263 ISSN: 1755-3245 * abstract, introduction * ----- | 1-10 | |
| A | HIGASHIDA H ET AL: "Sympathetic potentiation of cyclic ADP-ribose formation in rat cardiac myocytes." THE JOURNAL OF BIOLOGICAL CHEMISTRY 19 NOV 1999 LNKD- PUBMED:10559213, vol. 274, no. 47, 19 November 1999 (1999-11-19), pages 33348-33354, XP002599264 ISSN: 0021-9258 * abstract * ----- | 1-10 | TECHNICAL FIELDS SEARCHED (IPC) G01N |

The present search report has been drawn up for all claims

| Place of search | Date of completion of the search | Examiner |
|---|---|---|
| Munich | 3 September 2010 | Hoesel, Heidi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 30 5841

This annex lists the patent family members relating to the patent documents cited in the above-mentioned European search report.
The members are as contained in the European Patent Office EDP file on
The European Patent Office is in no way liable for these particulars which are merely given for the purpose of information.

03-09-2010

| Patent document cited in search report | Publication date | Patent family member(s) | | Publication date |
|---|---|---|---|---|
| WO 0140799 A2 | 07-06-2001 | AU | 4138601 A | 12-06-2001 |
| | | US | 2001008883 A1 | 19-07-2001 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0882169 A

**Non-patent literature cited in the description**

- **KRANIAS et al.** *Biochim. Biophys. Acta,* 1982, vol. 709, 28-37
- **ULLMAN et al.** *Proc. Natl. Acad. Sci., USA,* 1994, vol. 91, 5426-5430
- **GRAEFF et al.** *Biochemistry,* 1996, vol. 35, 379-386
- **ULLMAN et al.** *Proc. Natl. Acad. Sci., USA,* 1990, vol. 91, 5426-5430